# EUROPEAN PATENT APPLICATION

(11) **EP 2 269 650 A2**
(43) Date of publication of application: **05.01.2011**
(21) Application number: 10181143.8
(22) Date of filing: 03.08.2005
(51) Int. Cl.: A61K 47/08, A61K 31/355, A61K 31/375, A61K 31/07, A61K 31/59, A61P 29/00

(54) **Carrier for enteral administration**

(30) Priority: 03.08.2004 AU 2004904366; 07.04.2005 AU 2005901723
(62) Divisional of application: 05768314.6
(71) Applicant: Vital Health Sciences Pty Ltd., Melbourne, VIC 3000 (AU)
(72) Inventor: West, Simon, Michael, Williamstown, VIC 3016 (AU); Ogru, Esra, Wheelers Hill, VIC 3150 (AU)
(74) Representative: Vossius & Partner

(57) **Abstract**

The present invention relates to a method for improving the efficacy and transport of enterally administered biologically active compounds, said method comprising the step of combining the biologically active compound selected from the group consisting of nutritional supplements, vitamins, nutraceuticals, nutrients and other health supplements with a carrier comprising an effective amount of one or more phosphate derivatives of one or more electron transfer agents or one or more complexes of phosphate derivatives of one or more electron transfer agents,

## Description

### Field of the Invention

This invention relates to a carrier for use in the enteral administration of biologically active compounds.

### Background of the Invention

In this specification, where a document, act or item of knowledge is referred to or discussed, this reference or discussion is not to be taken as an admission that the document, act or item of knowledge was at the priority date: part of common general knowledge; or known to be relevant to an attempt to solve any problem with which this specification is concerned.

The major objective in drug delivery is to obtain an appropriate biological effect at a desired site of action. The choice of formulation can be critical to the efficacy of a drug since the bioactivity of a drug will be sub-optimal if it does not possess the correct physiochemical properties to allow release from the formulation at the target site of action.

Drugs are presented in different forms which are usually classified as enteral or parenteral. Enteral refers to forms of administration which involve entering the body through the gastrointestinal (***GI***) tract. Parenteral refers to all forms of administration which do not involve the GI tract. This classification highlights the fact that the GI tract introduces specific pharmacokinetic issues which do not arise with parenteral routes, in particular, first pass metabolism through the liver. Generally the form of administration differs markedly as illustrated in the following table.

| Enteral Administration | Parenteral Administration |
|---|---|
| Pills, tablets and capsules | Intranasal administration |
| Enteric-coated preparations | Subcutaneous Administration |
| Sustained-release preparations | Intramuscular injections |
| Oral liquid preparations | Intravenous injections |
| Bucchal / Sublingual administration | Topical preparations |
| Rectal administration | Transdermal administration |

Enteral delivery involves administering the drug via the GI tract where the drug is absorbed and distributed via the bloodstream to the target site of action. Drugs delivered orally are absorbed through the intestine, drugs delivered bucchally are absorbed through the mouth and drugs delivered rectally are absorbed through the rectum.

Oral delivery is dependent on the normal digestive processes of the GI tract. Once the oral drug formulation has been swallowed it travels through the esophagus to the stomach. The stomach has three tasks to do: (1) storage which requires the upper part of the stomach to relax and accept large volumes of swallowed material; (2) combine the swallowed material with digestive juice which requires the lower part of the stomach to mix these materials by its muscle action; and (3) empty its contents slowly into the small intestine. Several factors affect emptying of the stomach, including the nature of the food (mainly its fat and protein content) and the degree of muscle action of the emptying stomach and the small intestine. As the food is digested in the small intestine and dissolved into the juices from the pancreas, liver, and intestine, the contents of the intestine are mixed and pushed forward to allow further digestion. Finally, all of the digested nutrients are absorbed through the intestinal walls. The undigested materials are propelled into the colon and expelled. The normal digestive processes which control the rate of drug delivery to / removal from the target site are (1) when the stomach empties the drug into the small intestine and (2) the time spent in the intestinal tract before absorption. Unfortunately, these processes cannot be controlled. However, by instructing a patient to take a drug before, after or with food it is possible to optimise the effect of these processes.

The chemical environment of the GI tract is also important to oral drug delivery. The drug must be in a form which is stable at the different pH of the various parts of the GI tract. If the drug forms a non-absorbable complex or is degraded chemically or enzymatically then this will decrease absorption. The drug must also be in solution in the GI fluids to be absorbed. Sedimentation of the drug involves the drug forming solid particles and thus leaving the solution. Adsorption onto luminal solid particles involves solids adsorbing the drug, that is removing the drug from solution. Both sedimentation and adsorption decrease absorption of the drug. In many cases, degradation and complexation can be circumvented, or at least minimized, by chemical or formulation approaches so that they do not present a limitation to drug uptake.

Further, if a drug is absorbed through the intestinal or stomach wall, it then must pass through the liver. The liver is designed to eliminate foreign compounds from the body. As a result, a significant proportion of the drug (for example, 40-50%) may be metabolised and excreted before its reaches the bloodstream. It is possible to reduce the effect of the liver on enteral administration by having the drug absorbed through the lining of the mouth (bucchal / sublingual) or the lining of the rectum (suppositories), however these routes are not always appropriate.

The role of the drug formulation in the delivery of drug to the target site of action should not be ignored. With any drug it is possible to alter its bioavailability considerably by formulation modification. Since a drug must be in solution to be absorbed from the GI tract, you may expect the bioavailability of a drug to decrease in the order: solution > suspension > capsule > tablet > coated tablet. This order may not always be followed but it is a useful guide.

Attempts to improve the bioavailability of enterally administered biologically active compounds involve either the formation of prodrugs, for example morphine sulphate or the use of excipients which improve absorption. There is still a need for enteral formulations which further improve the bioavailability of biologically active compounds.

### Summary of the Invention

It has been found that a carrier composition comprising phosphates of electron transfer agents increases the efficacy of biologically active compounds administered enterally.

According to a first aspect of the invention, there is provided a carrier for use in enteral administration of biologically active compounds, said carrier comprising an effective amount of one or more phosphate derivatives of one or more electron transfer agents.

According to a second aspect of the invention, there is provided a method for improving the efficacy and transport of enterally administered biologically active compounds, said method comprising the step of combining the biologically active compound with a carrier comprising an effective amount of one or more phosphate derivatives of one or more electron transfer agents.

Preferably, the phosphate derivative of an electron transfer agent is selected from the group consisting of phosphate derivatives of tocopherol, phosphate derivatives of tocotrienol and mixtures thereof.

The present invention also provides for the use of an effective amount of one or more phosphate derivatives of one or more electron transfer agents, such as phosphate derivatives of tocopherol or tocotrienol, together with other excipients, in the manufacture of a carrier for use in the enteral administration of biologically active compounds.

The present invention also provides a pharmaceutical composition for enteral administration comprising one or more biologically active compounds and a carrier comprising an effective amount of one or more phosphate derivatives of one or more electron transfer agents, such as phosphate derivatives of tocopherol or tocotrienol.

According to a third aspect of the invention, there is provided a method for improving the efficacy and transport of enterally administered biologically active compounds, said method comprising the step of combining the biologically active compound with a carrier comprising an effective amount of one or more complexes of phosphate derivatives of an electron transfer agent.

According to a fourth aspect of the invention, there is provided a carrier for use in enteral administration of biologically active compounds, said carrier comprising an effective amount of one or more complexes of phosphate derivatives of an electron transfer agent.

In one preferred form of the invention, the carrier and biologically active compound are in a form which is protected by an enteric coating. The enteric coating must be insoluble in the stomach (low pH) and survive the enzymes in the saliva, but degrade in the absorption site which is just after the stomach at a pH greater than 6. Typically, the coating is a water soluble polymer such as cellulose ether, polyvinylpyrrolidone or polyethylene glycol. The advantage of the enteric coating is that it increases the likelihood that the carrier and the biologically active compound will be in proximity to each other at the absorption site and thus maximises the effects of the carrier upon the efficacy and transport of the biologically active compound. For example, a tablet or capsule may have an enteric coating or a functional food may contain microencapsulated particles which have an enteric coating.

The term "carrier" is used in this specification to include all forms of enteral administration. It includes but is not limited to pills, tablets, capsules, liquid formulations, functional foods, dietary supplements, lozenges, suppositories.

The term "effective amount" is used herein to refer to an amount of the one or more phosphate derivatives of an electron transfer agent that assists the biologically active compound to be absorbed in an amount that is measurably effective in the reduction of one or more symptoms presented by a patient. The effective amount may range up to 99.99% w/w of the total weight of the carrier. A person skilled in the art will understand that the actual amount will vary depending upon the biologically active compound. The effective amount will be sufficient to deliver an amount of biologically active compound within the therapeutic range of that biologically active compound. The effective amount used will also depend on whether the phosphate derivative of an electron transfer agent is being used to assist with formulation properties, for example, solubilisation or surface activity. Where the phosphate derivative of an electron transfer agent is acting as a solubiliser, the effective amount will depend on the concentration of the drug in the formulation and may range from 40% to 90% w/w, preferably 45 to 75% w/w, more preferably 50 to 60% w/w. Where the phosphate derivative of an electron transfer agent is not required for solubilisation properties, the effective amount may be in the range of 0.01 to 20% w/w, preferably 1 to 15% w/w and more preferably 5 to 10% w/w.

Preferably (when solubilisation properties are not required), the effective amount of the one or more phosphate derivatives of an electron transfer agent is in the range of from 0.1 to 10 % w/w of the total weight of the carrier. More preferably, in the range of 5 to 10% and most preferably 7.5% w/w.

The term "electron transfer agents" is used herein to refer to the class of chemicals which may be phosphorylated and which (in the non-phosphorylated form) can accept an electron to generate a relatively stable molecular radical or accept two electrons to allow the compound to participate in a reversible redox system. Examples of classes of electron transfer agent compounds that may be phosphorylated include hydroxy chromans including alpha, beta, gamma and delta tocols in enantiomeric and raecemic forms; quinols being the reduced forms of electron transfer agent K1 and ubiquinone; hydroxy carotenoids including retinol; calciferol and ascorbic acid. Preferably, the electron transfer agent is selected from the group consisting of tocopherol and other tocols, retinol, electron transfer agent K1 and mixtures thereof.

More preferably, the electron transfer agent is selected from the group consisting of the tocols and mixtures thereof. The tocols include all isomers of derivatives of 6:hydoxy 2:methyl chroman (see structure below) where R₁, R₂ and R₃ may be hydrogen or methyl groups, that is, the α-5:7:8 tri-methyl; β-5:8 di-methyl; γ-7:8 di-methyl; and δ 8 methyl derivatives. In the tocopherols, R₄ is substituted by 4:8:12 tri-methyl tridecane and the 2, 4, and 8 positions (see *) may be sterioisomers with R or S activity or racemic. In the tocotrienols, R₄ is substituted by 4:8:12 tri-methyl trideca-3:7:1 triene and the 2 position may be sterioactive as R or S sterioisomers or racemic. Most preferably, the electron transfer agent is α-tocopherol or tocotrienol.

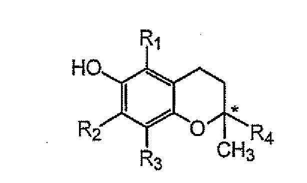

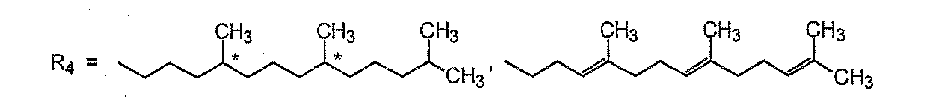

The term "phosphate derivatives" is used herein to refer to the acid forms of phosphorylated electron transfer agents, salts of the phosphates including metal salts such as sodium, magnesium, potassium and calcium and any other derivative where the phosphate proton is replaced by other substituents such as ethyl or methyl groups or phosphatidyl groups. The term includes mixtures of phosphate derivatives, especially those which result from phosphorylation reactions, as well as each of the phosphate derivatives alone. For example, the term includes a mixture of mono-tocopheryl phosphate (TP) and di-tocopheryl phosphate (T2P) as well as each of TP and T2P alone. Suitable mixtures are described in international patent application no PCT/AU01/01475.

Preferably, the one or more phosphate derivatives of one or more electron transfer agents is selected from the group consisting of mono-tocopheryl phosphate, di-tocopheryl phosphate, mono-tocotrienyl phosphate, di-tocotrienyl phosphate and mixtures thereof. Most preferably, the one or more phosphate derivatives of one or more electron transfer agents is a mixture of one or more of mono-tocopheryl phosphate, di-tocopheryl phosphate, mono-tocotrienyl phosphate and di-tocotrienyl phosphate.

In some situations, it may be necessary to use a phosphate derivative such as a phosphatide where additional properties such as increased water solubility are preferred. Phosphatidyl derivatives are amino alkyl derivatives of organic phosphates. These derivatives may be prepared from amines having a structure of R₁R₂N(CH₂)ₙOH wherein n is an integer between 1 and 6 and R₁ and R₂ may be either H or short alkyl chains with 3 or less carbons. R₁ and R₂ may be the same or different. The phosphatidyl derivatives are prepared by displacing the hydroxyl proton of the electron transfer agent with a phosphate entity that is then reacted with an amine, such as ethanolamine or N,N' dimethylethanolamine, to generate the phosphatidyl derivative of the electron transfer agent. One method of preparation of the phosphatidyl derivatives uses a basic solvent such as pyridine or triethylamine with phosphorous oxychloride to prepare the intermediate which is then reacted with the hydroxy group of the amine to produce the corresponding phosphatidyl derivative, such as P cholyl P tocopheryl dihydrogen phosphate.

In some situations, complexes of phosphate derivatives of the electron transfer agents may also be utilized where additional properties such as improved stability or deliverability may be useful. The term "complexes of phosphate derivatives" refers to the reaction product of one or more phosphate derivatives of electron transfer agents with one or more complexing agents selected from the group consisting of amphoteric surfactants, cationic surfactants, amino acids having nitrogen functional groups and proteins rich in these amino acids as disclosed in international patent application no PCT/AU01/01476, incorporated herein by reference.

The preferred complexing agents are selected from the group consisting of arginine, lysine and tertiary substituted amines, such as those according to the following formula:

NR¹R²R³

wherein R¹ is chosen from the group comprising straight or branched chain mixed alkyl radicals from C6 to C22 and carbonyl derivatives thereof;
R² and R³ are chosen independently from the group comprising H, CH₂COOX, CH₁CHOHCH₂SO₃X, CH₁CHOHCH₂OPO₃X, CH₂CH₂COOX, CH₂COOX, CH₂CH₂CHOHCH₂SO₃X or CH₂CH₂CHOHCH₂OPO₃X and X is H, Na, K or alkanolamine provided R² and R³ are not both H; and
wherein when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

Preferred complexing agents include arginine, lysine or lauryliminodipropionic acid where complexation occurs between the alkaline nitrogen centre and the phosphoric acid ester to form a stable complex.

The term "biologically active compound" is used herein to refer to compounds having a biological effect in humans or animals for medical or veterinary application. Biologically active compounds include pharmaceuticals, nutritional supplements, drugs, vitamins, phytochemicals, cosmeceuticals, nutraceuticals, nutrients and other health supplements that are useful for the treatment of human beings or other animals for prophylaxis or therapy. Examples of biologically active compounds include but are not limited to narcotic analgesics such as morphine and levorphanol, non narcotic analgesics such as codeine and acetaminophen, corticosteroids such as cortisone, anaesthetics such as propofol, antiemetics such as scopolamine, sympathomimetic drugs such as adrenaline and dopamine, antiepileptic drugs such as fosphenytoin, anti-inflammatory drugs such as ibuprofen, thyroid hormones and antithyroid drugs including thyroxine, phytochemicals including α-bisabolol, eugenol, silybin, soy isoflavones, iridoid gylcosides including aucubin and catalpol, sesquiterpene lactones including pseudoguaianolide from Arnica chamissonis, terpenes including rosmarinic acid and rosmanol, phenolic glycosides including the salicylates salicin, saligenin and salicyclic acid, triterpenes taxasterol or α-lactucerol, and isolactucerol, *p*-hydroxyphenylacetic acid derivative taraxacoside, hydroquinone derivatives including arbutin, phenylalkanones including gingerols and shagaols, hypercin, statins, and acylphloroglucides including xanthohumol, lupulone, humulone and 2-methylbut-3-en-2-ol. Examples of nutrients and nutraceuticals include vitamins, important precursor molecules for generation of hormones, minerals, proteins, minerals, amino acids, plant extracts such as grape seed extract, ephedrine, DHEA, isoflavones, phytosterols and similar bioagents. The biologically active compound may also be a peptide, polypeptide or protein. The biologically active compound can be in any suitable form including phosphate derivatives.

A person skilled in the art would know which other excipients could be included in the carrier. The choice of other excipients would depend on the characteristics of the biologically active compound. Examples of other excipients include solvents, surfactants, emollients, preservatives and the like. The choice of other excipients will also depend on the form of administration used.

### Brief Description of the Drawings

Figure 1: Effect of morphine sulphate 5 mg/kg, morphine with tocopheryl phosphate carrier according to the invention 5 mg/kg and control on paw withdrawal latency in rats, tested over 3 hours (pooled data).
Figure 2: CoQ₁₀ and CoQ₉ standard curves

The present invention is summarized by the following preferred embodiments:
1 A method for improving the efficacy and transport of enterally administered biologically active compounds, said method comprising the step of combining the biologically active compound with a carrier comprising an effective amount of one or more phosphate derivatives of one or more electron transfer agents.
2 The method according to item 1 wherein the electron transfer agent is selected from the group consisting of hydroxy chromans including alpha, beta, gamma and delta tocols in enantiomeric and raecemic forms; quinols being the reduced forms of electron transfer agent K1 and ubiquinone; hydroxy carotenoids including retinol; calciferol and ascorbic acid.
3 The method according to item 2 wherein the electron transfer agent is selected from the group consisting of tocopherol and other tocols, retinol, electron transfer agent K1 and mixtures thereof.
4 The method according to item 3 wherein the electron transfer agent is selected from the group consisting of tocols and mixtures thereof.
5 The method according to item 4 wherein the phosphate derivative of an electron transfer agent is selected from the group consisting of phosphate derivatives of tocopherol, phosphate derivatives of tocotrienol and mixtures thereof.
6 The method according to item 5 wherein the phosphate derivative of an electron transfer agent is selected from the group consisting of mono-tocopheryl phosphate, di-tocopheryl phosphate, mono-tocotrienyl phosphate, di-tocotrienyl phosphate and mixtures thereof.
7 The method according to item 1 wherein the phosphate derivative is selected from the group consisting of phosphates, phosphatides, complexes of phosphates and mixtures thereof.
8 The method according to item 1 further comprising the step of protecting the combined carrier and biologically active compound with an enteric coating.
9 The method according to item 1 wherein the biologically active compound is morphine.
10 The method according to item 1 wherein the biologically active compound is coenzyme Q10.
11 A carrier for use in enteral administration of biologically active compounds, said carrier comprising an effective amount of one or more phosphate derivatives of one or more electron transfer agents.
12 Use of an effective amount of one or more phosphate derivatives of one or more electron transfer agents, together with other excipients, in the manufacture of a carrier for use in the enteral administration of biologically active compounds.
13 A pharmaceutical composition for enteral administration comprising one or more biologically active compounds and a carrier comprising an effective amount of one or more phosphate derivatives of one or more electron transfer agents.
14 A method for improving the efficacy and transport of enterally administered biologically active compounds, said method comprising the step of combining the biologically active compound with a carrier comprising an effective amount of one or more complexes of phosphate derivatives of an electron transfer agent.
15 A carrier for use in enteral administration of biologically active compounds, said carrier comprising an effective amount of one or more complexes of phosphate derivatives of an electron transfer agent.

### Examples

The invention is further explained and illustrated by the following non-limiting examples.

### Example 1

In this experiment, the efficacy of a morphine composition according to the invention was compared with the efficacy of morphine sulphate, the currently used enteral formulation of morphine. The effect was measured by comparison of times taken for a rat to withdraw its paw in response to heat when medicated and unmedicated with morphine.

### Materials

Animals: Nine conscious Sprague-Dawley rats weighing between 350-450 grams each
Treatment groups:
1. Control: water,
2. morphine sulphate,
3. Morphine with TPm: morphine HCl (14%) in a carrier containing water (59%) and a tocopheryl phosphate mixture (27%) (***TPm***). The TPm contained mono-tocopheryl phosphate and di-tocopheryl phosphate.

Formulations 2 and 3 were diluted with water and the final morphine concentration was made up to 5 mg/ml. For example, 0.357 grams of formulation 3 was mixed with 0.643 grams of water to obtain a final morphine concentration of 5%. This liquid formulation was then delivered to the animals by oral gavage (tube into stomach).

### Method

The experiment used nine rats that were divided into three groups. After the first treatment, the rats were rested and each group was given a different treatment. The process was repeated once more until each rat had been given each of the three treatments.

Water, morphine sulphate and morphine with TPm were given by oral gavage at a concentration of 5mg/kg of body weight. Analgesic testing was performed at 1, 2, 4 and 6 hours and at each time point withdrawal latency was measured three times on each rat (with at least five minutes rest if using the same paw).

A plantar analgesiometer designed for rapid and efficient screening of analgesia levels in small laboratory animals was used. The device applied a heat source (∼45°C from an infrared light) to the animal's hindpaw and the time taken to withdraw the paw from the heat source was measured (paw withdrawal latency). The heat source (plate) provided a constant surface temperature. It had a built-in digital thermometer with an accuracy of 0.1°C and a timer with an accuracy of 0.1 second. The animal was placed on a hot plate, confined by a clear acrylic cage which surrounds the plate and paw withdrawal response was monitored. An increased time period before paw withdrawal response indicating analgesia. Each animal was tested 3 times at each time point. (ie a single rat had the heat applied to its back foot three times at each time point).

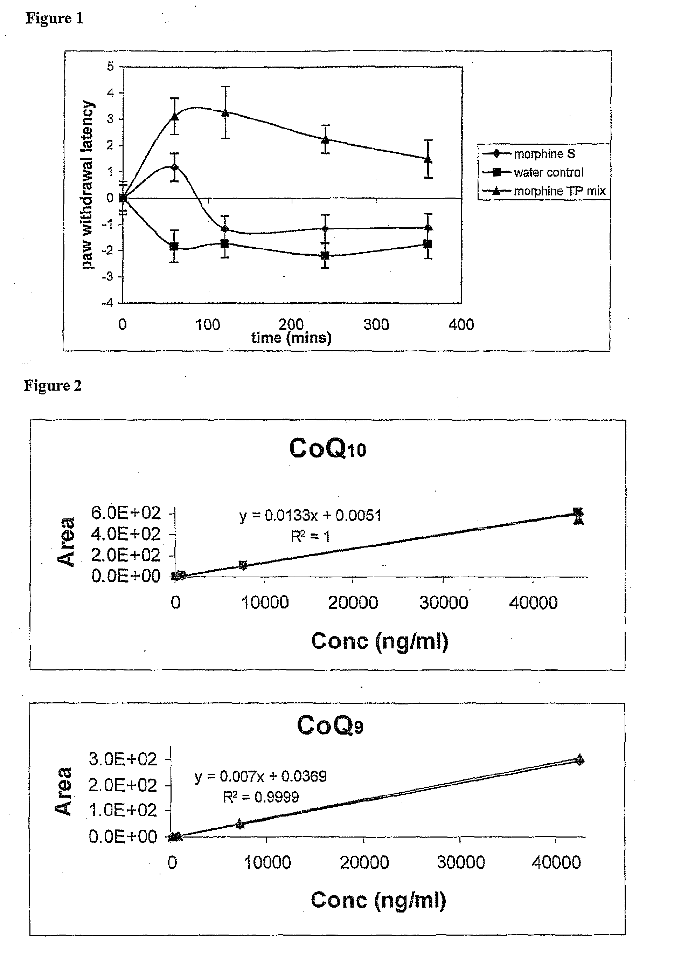

The results are illustrated in Figure 1. Both the morphine sulphate and morphine in the tocopheryl phosphate carrier caused an increase in latency indicating analgesia. The morphine in the tocopheryl phosphate carrier caused a greater latency which was maintained for a longer period of time than the morphine sulphate. That is, the morphine formulated in a carrier according to the invention provided a sustained analgesic effect for up to 2 hours following oral administration whereas the morphine sulphate only provided an analgesic effect for the first hour The standard error bars on the graph points do not overlap except at the one hour time point where the aqueous morphine sulphate and the morphine TPm formulation were similarly active. For the later time points, the morphine TPm formulation gave sustained analgesia.
1 Statistical Analysis: Comparison between morphine sulphate and Morphine TPm formulations.
   - at 60mins t=2,598 (p<0.02)
   - at 120mins t=4.815 (p<0.0005)
   - at 240mins t=4.351 (p<0.001)
   - at 360 mins t=3.094 (p=0.005)

### Conclusion

The use of the TPm carrier provided a sustained analgesia over a longer period of time using the same amount of morphine as the morphine sulphate formulation. Whilst the results were not significant at the one hour time point, the TPm formulation was statistically significant at all later time points.

### Example 2

This example investigates the bioavailability in guinea pigs of CoQ10 administered in the following formulations:
A. CoQsol
B. CoQsol plus TPM in MCT
C. MCT oil (control)

### Materials and Methods

### Formulations

Tocopheryl phosphate mixture (TPM) containing monotocopheryl phosphate (TP) and ditocopheryl phosphate (T2P) in a ratio of 2:1 w/w was prepared by Phosphagenics Ltd.

CoQso1 was purchased from Doctor's Trust Vitamins, U.S.A

Medium chain triglyceride (MCT) was manufactured by Abitec Corp, U.S.A.

The formulations consisted of the following:
A. CoQsol: Each softgel capsule contains 60 mg of CoQ, with the oily contents of the pills measuring 0.44 ml in volume. Therefore, the concentration of CoQ is 60 mg/0.44 ml = 136 mg CoQ/ml of capsule contents. Each millilitre of the CoQsol formulation also contains 136 IU d-α-tocopherol and 3705 IU vitamin A. Excipients are rice bran oil, gelatin, glycerin, water, beeswax, annato extract and titanium dioxide.
B. CoQsol+TPM: the formulation was prepared with the concentration of CoQ such that 30 mg/kg was administered in the same volume relative to body weight of the treatment group; i.e., in volumes of approximately 0.21 ml per kg b.wt. Each ml of formulation contained CoQ and TPM, each at 140 mg/ml, with MCT as the diluent.
C. MCT: (vehicle): the control group received MCT at 0.21 ml/kg b.wt.

### Animals

Adult female guinea pigs were purchased from Animal Services, Monash University and acclimatised to the Departmental Animal House for a minimum of 5 days before the treatments commenced. Animals were randomly assigned to treatment groups (n=10), tagged with unique identifying marks on their backs (clipped hair and colour codes), and housed as a group in an environmentally-enriched pen of approximately 1 x 4 m in size. The average body weight of the CoQsol-treated group was 0.795 kg at day 0. The average body weight of the CoQso1+TPM group was 0.746 kg at day 0. The average body weight of the control group was 0.796kg at day 0.

Food and water: Rabbit and guinea pig standard laboratory pellets (Barastoc, Australia). Water was provided freely

Route and method of dosing: animals were dosed by oral gavage using a plastic cannula attached to a delivery syringe in volumes of approximately 0.21 ml per kg body weight.

### Methods

Dose of CoQsol: 30 mg / kg body weight / day

Dose of TPM: 30 mg / kg body weight / day

Dosing regimen: once daily

Dosing period: 26 days

Body weight was measured weekly.

At the completion of the treatment period, the guinea pigs were killed by asphyxiation using CO₂ gas. The blood was removed by heart puncture into heparinised collection tubes, and centrifuged for separation of plasma and stored at -80°C until extraction of CoQ.

Extractions of CoQ and analyses by HPLC were performed essentially according to the method of Aberg et al., (1992) "Distribution and redox state of ubiquinones in rat and human tissues" Arch. Biochem. Biophys. 295: 230-34.

Increased levels of CoQ10 and CoQ9 are indicative of increased bioavailability and uptake. Both CoQ9 and CoQ10 were measured because guinea pigs can synthesize both formes of CoQ (9 and 10). It is therefore important to assess the levels of both forms following administration since administration of CoQ10 can increase both levels in vivo.

### Results:

CoQ₁₀ and CoQ₉ concentration in plasma

| | **CoQ₉** | | | | | |
|---|---|---|---|---|---|---|
| | **Peak** | **CoQ₁₀** | **CoQ₉ Conc** | **CoQ₁₀ Cone** | **CoQ₉ Cone** | **CoQ₁₀ Conc** |
| **PLASMA Control** | **area** | **Peak area** | **(ng/ml)** | **(ng/ml)** | **(ng/vial)** | **(ng/vial)** |
| PlasmaC1 | 0.191146 | 3.89095 | 22 | 292 | 11 | 146 |
| PlasmaC2 | 1.26553 | 2.62247 | 176 | 197 | 88 | 98 |
| PlasmaC3 | 0.69327 | 1.15616 | 94 | 87 | 47 | 43 |
| PlasmaC4 | 0.874554 | 2.29567 | 120 | 172 | 60 | 86 |
| PlasmaC5 | 0.558185 | 2.45003 | 74 | 184 | 37 | 92 |
| PlasmaC6 | 0.90064 | 5.16449 | 123 | 388 | 62 | 194 |
| PlasmaC7 | 1.0674 | 3.34757 | 147 | 251 | 74 | 126 |
| PlasmaC8 | 0.190272 | 3.20794 | 22 | 241 | 11 | 120 |
| PlasmaC9 | | 3.18797 | | 239 | | 120 |
| PlasmaC10 | | 1.14622 | | 86 | | 43 |
| **Mean** | | | | | 49 | 107 |
| PlasmaS1 | 0.902389 | 5.18353 | 124 | 389 | 62 | 195 |
| PlasmaS2 | 0.19846 | 2.45755 | 23 | 184 | 12 | 92 |
| PlasmaS3 | 0.32906 | 2.60398 | 42 | 195 | 21 | 98 |
| PlasmaS4 | 0.45307 | 3.0014 | 59 | 225 | 30 | 113 |
| PlasmaS5 | 0.53315 | 2.3862 | 71 | 179 | 35 | 90 |
| PlasmaS6 | 0.93297 | 2.04795 | 128 | 154 | 64 | 77 |
| PlasmaS7 | 0.54571 | 2.1518 | 73 | 161 | 36 | 81 |
| PlasmaS8 | 1.41097 | 2.16559 | 196 | 162 | 98 | 81 |
| PlasmaS9 | 0.690034 | 6.3554 | 93 | 477 | 47 | 239 |
| PlasmaS10 | 0.28032 | 3.61253 | 35 | 271 | 17 | 136 |
| **Mean** | | | | | 42 | 120 |

| **CoQsol+TPM** | | | | | | |
|---|---|---|---|---|---|---|
| PlasmaT1 | 0.84019 | 2.75545 | 115 | 207 | 57 | 103 |
| PlasmaT2 | | 4.47825 | | 336 | | 168 |
| PlasmaT3 | 0.17022 | 4.3493 | 19 | 327 | 10 | 163 |
| PlasmaT4 | 0.503096 | 4.92475 | 67 | 370 | 33 | 185 |
| PlasmaT5 | 0.372346 | 3.01239 | 48 | 226 | 24 | 113 |
| PlasmaT6 | 0.267389 | 6.29373 | 33 | 473 | 16 | 236 |
| PlasmaT7 | | 5.53598 | | 416 | c | 208 |
| PlasmaT8 | 0.26282 | 5.51973 | 32 | 415 | 16 | 207 |
| PlasmaT9 | 0.336923 | 4.1363 | 43 | 311 | 21 | 155 |
| PlasmaT10 | 0.105107 | 3.49962 | 10 | 263 | 5 | 131 |
| **Mean** | | | | | 23 | 167 |

The above results do not include any statistical information because as is known in the area of micronutrient studies, there is always a significant inter-animal variation due to the fact that each animal has different needs for CoQ10 and it will only be absorbed when needed. The large inter-animal variation leads to large standard deviation figures, which do not accurately reflect the significance of the results.

Figure 2 shows the standard curves obtained in the analysis of CoQ10 and CoQ9 in the plasma before running the samples on the HPLC (the closer r² is to 1 the more accurate the results).

### Discussion

The results in Figure 2 and the above tables show that the CoQ levels in plasma are higher in CoQsol+TPM treated animals than in the animals treated with CoQsol alone or the control. This illustrates that the tocopheryl phosphate mixture increases the bioavailability of CoQ10. The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A method for improving the efficacy and transport of enterally administered biologically active compounds, said method comprising the step of combining the biologically active compound selected from the group consisting of nutritional supplements, vitamins, nutraceuticals, nutrients and other health supplements with a carrier comprising an effective amount of one or more phosphate derivatives of one or more electron transfer agents or one or more complexes of phosphate derivatives of one or more electron transfer agents, wherein the electron transfer agent is selected from the group consisting of hydroxy chromans including alpha-, beta-, gamma- and delta-tocols in enantiomeric and racemic forms; quinols being the reduced forms of electron transfer agent K1 and ubiquinone; hydroxy carotenoids including retinol; calciferol and ascorbic acid and wherein the phosphate derivative is selected from the group consisting of phosphates, phosphatides, complexes of phosphates and mixtures thereof; and wherein "complexes of phosphate derivatives" refers to the reaction product of one or more phosphate derivatives of electron transfer agents with one or more complexing agents selected from the group consisting of amphoteric surfactants, cationic surfactants, amino acids having nitrogen functional groups and proteins rich in these amino acids.

2. The method according to claim 2 wherein the electron transfer agent is selected from the group consisting of tocopherol and other tocols, retinol, electron transfer agent K1 and mixtures thereof.

3. The method according to claim 3 wherein the electron transfer agent is selected from the group consisting of tocols and mixtures thereof.

4. The method according to claim 4 wherein the phosphate derivative of an electron transfer agent is selected from the group consisting of phosphate derivatives of tocopherol, phosphate derivatives of tocotrienol and mixtures thereof.

5. The method according to claim 5 wherein the phosphate derivative of an electron transfer agent is selected from the group consisting of mono-tocopheryl phosphate, di-tocopheryl phosphate, mono-tocotrienyl phosphate, di-tocotrienyl phosphate and mixtures thereof.

6. The method according to claim 1 wherein the mixture of phosphate derivatives of an electron transfer agent is a mixture of mono-tocopheryl phosphate and di-tocopheryl phosphate.

7. The method according to claim 1 further comprising the step of protecting the combined carrier and biologically active compound with an enteric coating.

8. The method according to claim 1 wherein the combined carrier and biologically active compound is in the form of a pill, tablet, capsule, liquid formulation, functional food, dietary supplement, lozenge or suppository.

9. A composition for enteral administration comprising one or more biologically active compounds selected from the group consisting of nutritional supplements, vitamins, nutraceuticals, nutrients and other health supplements and a carrier comprising an effective amount of one or more phosphate derivatives of one or more electron transfer agentsor one or more complexes of phosphate derivatives of one or more electron transfer agents, wherein the electron transfer agent is selected from the group consisting of hydroxy chromans including alpha-, beta-, gamma- and delta-tocols in enantiomeric and racemic forms; quinols being the reduced forms of electron transfer agent K1 and ubiquinone; hydroxy carotenoids including retinol; calciferol and ascorbic acid and wherein the phosphate derivative is selected from the group consisting of phosphates, phosphatides, complexes of phosphates and mixtures thereof; and wherein "complexes of phosphate derivatives" refers to the reaction product of one or more phosphate derivatives of electron transfer agents with one or more complexing agents selected from the group consisting of amphoteric surfactants, cationic surfactants, amino acids having nitrogen functional groups and proteins rich in these amino acids.

10. A composition which is a pharmaceutical composition.
